(19) Europäisches Patentamt / European Patent Office / Office européen des brevets

(11) EP 1 692 284 B1

(12) EUROPÄISCHE PATENTSCHRIFT

(45) Veröffentlichungstag und Bekanntmachung des Hinweises auf die Patenterteilung:
**02.07.2008 Patentblatt 2008/27**

(21) Anmeldenummer: **04803742.8**

(22) Anmeldetag: **10.12.2004**

(51) Int Cl.:
*C12N 15/11* (2006.01) *C12Q 1/68* (2006.01)
*A61K 48/00* (2006.01)

(86) Internationale Anmeldenummer:
**PCT/EP2004/014097**

(87) Internationale Veröffentlichungsnummer:
**WO 2005/056793 (23.06.2005 Gazette 2005/25)**

(54) **ANTI-APOPTOTISCH WIRKSAME APATAMERE**

ANTI-APOPTOTICALLY ACTIVE APATAMERS

APATAMERES A ACTION ANTI-APOPTOTIQUE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **11.12.2003 DE 10358407**

(43) Veröffentlichungstag der Anmeldung:
**23.08.2006 Patentblatt 2006/34**

(73) Patentinhaber: **CytoTools GmbH**
**64285 Darmstadt (DE)**

(72) Erfinder:
- **KLOCK, Gerd**
  **64807 Dieburg (DE)**
- **FREYBERG, Mark, Andre**
  **64287 Darmstadt (DE)**
- **KAISER, Dirk**
  **64859 Eppertshausen (DE)**

(56) Entgegenhaltungen:
**WO-A-01/33218** **WO-A-02/26932**
**WO-A-02/083160**

- **FREYBERG M A ET AL: "Proatherogenic flow conditions initiate endothelial apoptosis via thrombospondin-1 and the integrin-associated protein" BIOCHEMICAL AND BIOPHYSICAL RESEARCH COMMUNICATIONS, Bd. 286, Nr. 1, 10. August 2001 (2001-08-10), Seiten 141-149, XP002324346 ISSN: 0006-291X in der Anmeldung erwähnt**
- **CERCHIA L ET AL: "Nucleic acid aptamers in cancer medicine" FEBS LETTERS, Bd. 528, Nr. 1-3, 25. September 2002 (2002-09-25), Seiten 12-16, XP004383225 ISSN: 0014-5793**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft anti-apoptotisch wirksame Aptamere. Die entsprechenden Sequenzen werden gezeigt. Mögliche therapeutische und diagnostische Verwendungen werden beschrieben.

Nukleinsäuremoleküle (sogenannte Aptamere), die spezifisch an bestimmte Zielmoleküle (Antigene) binden, sowie Verfahren zur Herstellung und Isolierung solcher Aptamere wurden im Stand der Technik bereits sehr ausführlich beschrieben.

Solche aus einzelsträngiger ssDNA oder ssRNA bestehenden Aptamere besitzen dabei sehr hohe Affinitäten und Spezifitäten für die entsprechenden Antigene. Bis heute wurden Nukleinsäureliganden für Metallionen, organische Verbindungen, Peptide, Proteine, oder sogar komplexe Strukturen wie Viren und Zellen isoliert (Übersichtsartikel: Gold et.al., Annu. Rev. Biochem. 64, (1995), 763-797; Ellington und Conrad, Biotechnol. Annu. Rev. 1 (1995), 185-214; Famulok, Curr. Opin. Struct. Biol. 9 (1999), 324-329).

Aptamere sind daher Nukleinsäuren, bestehend aus DNA oder RNA, die durch ihre räumliche Struktur in der Lage sind, spezifisch und mit hoher Affinität an ein bestimmtes Target zu binden (Osborne, S. E. and A. D. Ellington (1997) "Nucleic Acid Selection and the Challenge of Combinatorial Chemistry" Chem. Rev 97(2): 349-370). In den letzten Jahren wurde eine Reihe von Aptameren gegen medizinisch relevante Zielproteine identifiziert, und es wird erwartet, dass der Einsatz von Nukleinsäuren für therapeutische und diagnostische Zwecke jetzt erst richtig beginnt (Jayasena, S. D., (1999), "Aptamers: an emerging class of molecules that rival antibodies in diagnostics" Clin Chem 45(9): 1628-1650, Cerchia, L., J. Hamm, et al., (2002), "Nucleic acid aptamers in cancer medicine" FEBS Lett 528(1-3): 12-16). Diese neue Entwicklung liegt darin begründet, dass die Identifizierung und Anwendung von DNA oder RNA-Molekülen einige Vorteile gegenüber Antikörpern aufweist, die Therapie- und Diagnose auf vielen Gebieten bisher noch dominieren.

Die Methodik zur Gewinnung monoklonaler Antikörper (Köhler, G. and C. Milstein (1975) "Continuous cultures of fused cells secreting antibody of predefined specificity" Nature 256 (5517): 495-497) bedeutete einen großen Durchbruch für viele Bereiche der modernen Biologie und Medizin. In der medizinischen Forschung sind sie vor allem auf diagnostischem Gebiet von Bedeutung, inzwischen sind einzelne Antikörper auch als Medikamente zugelassen worden. Wenn ein bestimmtes Protein, z.B. an der Zelloberfläche, als Target für diagnostische oder therapeutische Zwecke identifiziert wurde und dann a) seine Präsenz diagnostisch nachgewiesen, oder b) seine Funktion therapeutisch blockiert werden sollte, so war bisher für beide Fälle die Entwicklung eines monoklonalen Antikörpers die wichtigste und/oder die schnellste Methode; die Entwicklung niedermolekularer Therapeutika ist immer noch sehr langwierig. Die Nachteile und Einschränkungen bei der Identifizierung und Produktion der Antikörper sind allerdings bekannt (Jayasena 1999), z.B. die Notwendigkeit, mit Tierversuchen die erste Immunisierung durchzuführen; das Problem der Verfügbarkeit und Reproduzierbarkeit der Hybridoma-Zellen über mehrere Jahre; oder der hohe Aufwand und die enormen Kosten bei der Herstellung der Antikörper.

Ein Aptamer kann unter spezifisch ausgewählten Versuchsbedingungen selektiert werden, wie z.B. solche, die für ein diagnostisches Verfahren optimal sind (Jayasena 1999). Aptamere können besser über längere Zeit gelagert werden, da sie nicht wie Proteine einer irreversiblen Denaturierung unterliegen, sondern jederzeit wieder thermisch renaturiert werden können. Aptamere werden während des Selektionsverfahrens meist enzymatisch synthetisiert, sie lassen sich für eine Produktion im größeren Maßstab aber auch chemisch synthetisieren, so dass die Herstellung gegenüber der Produktion von monoklonalen Antikörpern erheblich besser reproduzierbar erfolgen kann (Jayasena 1999).

Die Modifizierungen, die zur Stabilisierung von DNA (Agrawal, S. (1996) "Antisense oligonucleotides: towards clinical trials" Trends Biotechnol 14(10): 376-387) oder RNA (Pieken, W. A., Olsen D. B. et al. (1991). "Kinetic characterization of ribonuclease-resistant 2'-modified hammerhead ribozymes." Science 253(5017): 314-317; Ruckman, J., Green L. S., et al. (1998) "2'-Fluoropyrimidine RNA-based aptamers to the 165-amino acid form of vascular endothelial growth factor (VEGF165). Inhibition of receptor binding and VEGFinduced vascular permeability through interactions requiring the exon 7-encoded domain." J Biol Chem 273(32): 20556-20567) führen, sind als Routineverfahren In der WO02083160 werden Peptide als Medikamente offenbart, die die Apoptose in Epithelzellen und Keratinozyten inhibieren, weil sie die Bindung von Thrombospondin-1 an IAP und/oder $\alpha_v\beta_3$ inhibieren. Solche Peptide können zur Wundheilung eingesetzt werden. in der Synthese der Nukleinsäuren bzw. ihrer Nukleotid-Vorstufen etabliert, so dass jetzt die ersten Aptamere in klinischen Studien getestet werden können (Eyetech Study Group (2002) "Preclinical and phase 1A clinical evaluation of an anti-VEGF pegylated aptamer (EYE001) for the treatment of exudative agerelated macular degeneration" Retina 22(2): 143-152).

Die Apoptose ist ein genetisch kodiertes Selbstmordprogramm, welches in eukaryontischen Zellen unter bestimmten physiologischen oder pathologischen Bedingungen induziert wird. Die Induktion der Apoptose muss außerordentlich präzise reguliert sein, denn eine Hyperaktivität kann zu degenerativen Erkrankungen führen. Auf der anderen Seite kann eine verringerte Apoptose-Induktion beispielsweise zur Tumorprogression beitragen.

Verschiedene niedermolekulare Induktoren der Apoptose wurden bereits beschrieben. Eine wichtige Klasse sind Tumorzytostatika. Auf welche Weise diese Zytostatika oder andere Substanzen Apoptose induzieren können, ist in den meisten Fällen jedoch unbekannt.

Die Induktion von Apoptose kann beispielsweise über eine Reihe von sog. Todesrezeptoren, d. h. Rezeptoren, die eine "Death Domain" (DD) enthalten, wie CD95, TNF-RI, DR3, DR4 oder DR5, erfolgen, die nach Bindung ihrer Liganden Apoptose-Signalwege induzieren. Beispielsweise interagiert nach Bindung des CD95-Liganden der CD95-Rezeptor mit dem Adapterprotein FADD/MORT1, wodurch das "Recruitment" und die Aktivierung der Protease FLICE/Caspase-8, am DISC "Death Inducing Signalling Complex" induziert werden. FADD und FLICE enthalten jeweils "Death Effector Domains" (DED). Die Induktion der Apoptose über diese Apoptose-Signalwege ist von außen beispielsweise durch die Gabe von Zellgiften (cytotoxischen Substanzen), Bestrahlung, Viren, Entzug von Wachstumsfaktoren oder mechanische Zellverletzung möglich. Diese Möglichkeiten der Apoptose-Tnduktion sind allerdings von bestimmten Nachteilen begleitet. So führt die Gabe von Zellgiften, wie Cytostatika, oder die Bestrahlung bei Krebszellen zur Resistenzentwicklung und darüber hinaus zu einer Schädigung normaler Zellen, bei denen eigentlich keine Apoptose ausgelöst werden sollte.

Im allgemeinen wird z.B. die Induktion der Apoptose vorgeschlagen zur Behandlung von Krebs, zur Verhinderung von angiogenetischen Vorgängen etc. Obwohl hier bereits Induktoren beschrieben wurden, weisen diese noch immer eine Reihe von Nachteilen auf. Beispielsweise erzeugen Zytostatika schwere Nebenwirkungen.

Es werden aber auch pathologische Zustände diskutiert, bei denen die Apoptose negative Auswirkungen hat, und zu deren Behandlung die Apoptose inhibiert werden muß.

Ein Beispiel für eine solche Krankheit ist die Arteriosklerose. Insbesondere konnte von den Erfindern schon früher gezeigt werden, dass gerade im Bereich der arteriosklerotischen Plaques apoptotische Zellen (besonders: Endothelzellen, glatte Muskelzellen) auftreten, und dass dieses Auftreten durch gestörte Strömungsverhältnisse verstärkt wird, also strömungsabhängig ist (Freyberg et al., BBRC, 286, 141-149, 2001).

Auch können solche Substanzen, die die Apoptose modulieren eingesetzt werden, um die Heilung von Wunden positiv zu beeinflussen. Weitere Krankheiten, die in Zusammenhang mit einer verstärkt auftretenden Apoptose diskutiert werden, sind AIDS, Krebs und Alzheimer. Weiterhin in Zusammenhang mit einer verstärkt auftretenden Apoptose diskutierte Erkrankungen sind systemischer Lupus erythematosus sowie die rheumatoide Arthritis sowie weitere chronisch-entzündliche Erkrankungen.

Es besteht daher ein hoher Bedarf an Substanzen, die die Apoptose positiv oder negativ beeinflussen. Im Sinne der vorliegenden Erfindung sind insbesondere solche Substanzen bevorzugt, die Apoptose inhibieren. Insbesondere wäre es günstig, die strömungsabhängige/kraftabhängige und mit der Arteriosklerose/Wundheilung ursächlich verbundene Apoptose inhibieren zu können. Weiterhin besteht hoher Bedarf an pharmazeutischen Formulierungen, die solche Substanzen enthalten, und die zur Behandlung von Zuständen eingesetzt werden können, bei denen die Inhibition oder die Induktion der Apoptose angezeigt ist, insbesondere zur Behandlung der Arteriosklerose, die positive Beeinflussung der Wundheilung, aber auch von AIDS, Alzheimer und Krebs.

In der älteren Deutschen Patentanmeldung DE 101 63 130 offenbaren die Erfinder der vorliegenden Erfindung Peptide, die Apoptose inhibieren oder induzieren.

Aufgabe der vorliegenden Erfindung war es daher, weiter verbesserte apoptotisch wirksame Substanzen zur Verfügung zu stellen. Insbesondere war es eine weitere Aufgabe der vorliegenden Erfindung, Substanzen zur Verfügung zu stellen, die eine durch TSP-1 induzierte Apoptose von eukarytischen Zellen, insbesondere von Endothelzellen und Fibroblasten inhibieren können. Eine weitere Aufgabe der vorliegenden Erfindung war es, pharmazeutische Zubereitungen zur Verfügung zu stellen, mit denen Krankheiten wie Arteriosklerose, Wunden, AIDS, Alzheimer, Krebs, systemischer Lupus erythematosus sowie die rheumatoide Arthritis sowie weitere chronisch-entzündliche Erkrankungen behandelt werden können, bei denen die Inhibition oder Induktion von. Apoptose angezeigt ist.

Diese und weitere, nicht explizit genannte Aufgaben, die sich aber aus der vorangegangenen Würdigung des Stands der Technik ohne weiteres ergeben, werden durch die in den Ansprüchen definierten Ausführungsbeispiele der vorliegenden Erfindung gelöst

Überraschenderweise kann diese Aufgabe in einfacher Weise gelöst werden durch zur Verfügung stellen einer Nukleinsäure umfassend eine Nukleinsäuresequenz ausgewählt aus der Gruppe bestehend aus den in den SEQ ID NOs 1 bis 9 dargestellten Sequenzen.

Ein weiteres Ausführungsbeispiel der vorliegenden Erfindung betrifft funktionelle Varianten dieser Nukleinsäuren. Damit sind für die Zwecke der vorliegenden Erfindung Nukleinsäuren gemeint, die mindestens 95% Sequenzidentität zu den Sequenzen ausgewählt aus der Gruppe bestehend aus: SEQ ID NO 1 bis SEQ ID NO 9 aufweisen, und in dem unten beschriebenen Testverfahren eine anti-apoptotische Aktivität von mindestens 50% Inhibitionsindex, bevorzugt mindestens 60%, besonders bevorzugt mindestens 70%, ganz besonders bevorzugt mindestens 80%, noch mehr bevorzugt mindestens 90% und am meisten bevorzugt mindestens 95% aufweisen.

Ein weiteres Ausführungsbeispiel der vorliegenden Erfindung betrifft Nukleinsäuren bei denen sich an eine Nukleinsäuresequenz ausgewählt aus der Gruppe bestehend aus: SEQ ID NO 1 bis SEQ ID NO 9 in 5'- und/oder 3'-Richtung Nukleotide anschliessen. Insbesondere umfasst diese Verlängerung jeweils nicht mehr als 100, bevorzugt nicht mehr als 70, besonders bevorzugt nicht mehr als 30, ganz besonders bevorzugt nicht mehr als 20, und am meisten bevorzugt nicht mehr als 10 Nukleotide.

Selbstverständlich sind auch hier funktionelle Varianten dieser verlängerten Nukleinsäuren im Sinne der obigen Definition mitumfasst.

Die Aptamere der vorliegenden Erfindung umfassen vorzugsweise Modifikationen, die die Stabilität der erfindungsgemäßen Nukleinsäuren gegenüber einem Nuklease-Abbau erhöhen. Bevorzugt handelt es sich dabei um die Verwendung von 2'-$NH_2$-2'-desoxyuridin und/oder 2'-$NH_2$-2'-desoxycytidin anstelle der unmodifizierten Nukleotide Uridin und Cytidin.

Unter Nukleinsäuren werden für die Zwecke der vorliegenden Erfindung polymere Moleküle verstanden, die im Falle der RNA aus den Nukleotiden Adenosin (A), Cytidin (C), Uridin (U), Guanosin (G) und die im Falle der DNA aus Desoxyadenosin (A), Desoxycytidin (C), Desoxyguanosin (G) und Thymidin (T) aufgebaut sind. Diese Nukleotide können mindestens eine der folgenden Modifikationen aufweisen: 2'-desoxy, 2'-Fluor, 2'-Chlor, 2'-Brom, 2'-Iod, 2'-Amino (bevorzugt nicht substituiert, mono- oder di-substituiert), 2'-mono-, di-oder tri-halomethyl, 2'-O-Alkyl, 2'-O-halo-substituiertes Alkyl, 2'-Alkyl, Azido, Phosphorothioat, Sulfhydryl, Methylphosphonat, Fluorescein, Rhodamin, Pyren, Biotin, Xanthin, Hypoxanthin, 2,6-Diaminopurin, 2-Hydroxy-6-mercaptopurin, Polyethylenglykol-Modifikationen und bei Pyrimidin-Basen an 6-Position Schwefel bzw. an 5-Position Halogen, $C_{1-5}$ Alkylgruppen, abasischer Linker, 3'-desoxy-adenosin oder andere "Kettenterminatoren" bzw. nicht-verlängerbare Nukleotidanaloge am 3'Ende oder andere Modifizierungen am 5'- oder/und 3'-Ende aufweisen. Weitere im Stand der Technik bereits offenbarte Modifikationen wie z.B. in der WO 02/26932 sind hier selbstverständlich mit umfasst.

Im allgemeinen umfassen Aptamere 10 bis 100 Nukleotide, vorzugsweise 15 bis 50 Nukleotide, besonders bevorzugt 20 bis 40 Nukleotide. Gegebenenfalls können Aptamere eine Minimalsequenz von etwa mindestens 6, bevorzugt etwa mindestens 10 und besonders bevorzugt mindestens 14 bis 15 Nukleotiden aufweisen, die nötig ist, um die Bindung zu gewährleisten.

Ein bevorzugtes Ausführungsbeispiel der vorliegenden Erfindung stellen pharmazeutische Zubereitungen umfassend mindestens eine erfindungsgemäße Nukleinsäure dar.

Ein insbesondere bevorzugtes Ausführungsbeispiel der vorliegenden Erfindung ist die Verwendung der erfindungsgemäßen Nukleinsäuren zur Herstellung eines Medikamentes zur Behandlung von Arteriosklerose, zur Förderung der Wundheilung, insbesondere bei schwerheilenden Wunden, AIDS, Krebs, Alzheimers Erkrankung, systemischer Lupus erythematosus sowie die rheumatoide Arthritis und weiterer chronisch-entzündlicher Erkrankungen mittels der im Fachgebiet gut bekannten Methoden.

In einem weiteren bevorzugten Ausführungsbeispiel betrifft die vorliegende Erfindung auch die Verwendung der erfindungsgemäßen Nukleinsäuren als Diagnosetool. Dabei werden markierte Nukleinsäuren verwendet. Die Markierung kann z.B. aus einem Fluoreszensfarbstoff, einem Enzym, Antikörper oder einem Radionuklid bestehen. Die entsprechenden Nachweismethoden sind dem Fachmann bestens bekannt.

Vorzugsweise wird ein erfindungsgmäßes Aptamer dann als Bestandteil eines kit of parts vorliegen nebst beispielsweise Positiv- und Negativkontrollen. Es ist dem Fachmann klar, daß die entsprechenden Aptamere insgesamt prinzipiell wie Antikörper z.B. in ELISA-Applikationen, chromatographischen Verfahren und für diagnostische/Screening-Verfahren angewendet werden können.

Anti-apoptotisch wirksam im Sinne der vorliegenden Erfindung bedeutet, dass die entsprechende Substanz in dem Inhibitionstest gemäß Beispiel 6 einen Inhibitions-Index von mindestens 50%, bevorzugt mindestens 60%, besonders bevorzugt mindestens 70%, ganz besonders bevorzugt mindestens 80%, noch mehr bevorzugt mindestens 90% und am meisten bevorzugt mindestens 95% bezogen auf die Kontrolle mit TSP-1-induzierter Apoptose bewirkt.

Aptamere, die aktive Bestandteile einer pharmazeutischen Zubereitung sind, werden im Allgemeinen in einem pharmazeutisch annehmbaren Träger gelöst. Beispiele für pharmazeutisch annehmbare Träger können Pufferlösungen wie Phosphatpuffer oder Citratpuffer sein.

Die spezifische Dosierung und Posologie ist für jeden Patienten von einer Anzahl von Faktoren abhängig, einschließlich der Aktivität der verwendeten spezifischen Verbindungen, dem Alter des Patienten, dem Körpergewicht, dem allgemeinen Gesundheitszustand, dem Geschlecht, der Ernährung, der Zeit der Verabreichung, dem Weg der Verabreichung, der Exkretionsrate, der Verbindung mit anderen Arzneimitteln und der Schwere der einzelnen Erkrankung, für die die Therapie angewandt wird. Sie wird von einem Arzt in Abhängigkeit von diesen Faktoren ermittelt.

Aptamermedikamente werden üblicherweise parenteral verabreicht, z.B. durch ein Inhalationsspray, rektal, durch subkutane, intravenöse, intramuskuläre, intraartikuläre und intrathecale Injektions- und Infusionstechniken, oder äußerlich in pharmazeutischen Formulierungen, welche konventionelle, pharmazeutisch annehmbare Träger, Adjuvantien und Vehikel enthalten. Je nach Art der identifizierten Substanz kommen auch andere Verabreichungswege in Betracht, z.B. oral. Weiterhin kann das Medikament als Salbe, Gel, feuchter Verband etc. aufgebracht werden.

Die Erfindung stellt ebenso pharmazeutische Zusammensetzungen bereit, die eine wirksame Menge eines anti-apoptotischen wirksamen Aptamers in Kombination mit einem konventionellen pharmazeutischen Träger umfassen. Ein pharmazeutischer Träger ist z.B. ein fester oder flüssiger Füllstoff, ein Verkapselungsmaterial oder ein Lösungsmittel. Beispiele für Materialien, die als pharmazeutische Träger dienen können, sind Zucker wie Lactose, Glucose und Saccharose; Stärke wie Maisstärke und Kartoffelstärke; Cellulose und deren Derivate wie Natriumcarboxymethylcellulose, Ethylcellulose und Celluloseacetat; pulverisierter Tragacanth; Malz; Gelatine; Talg; Arzneimittelträger wie Kakaobutter und Zäpfchenwachse; Öle wie Erdnussöl, Baumwollsamenöl, Färberdistelöl, Sesamöl, Olivenöl, Maisöl und Sojabohnenöl; Polyole wie Propylenglykol, Glycerin, Sorbitol, Mannitol und Polyethylenglykol; Ester wie Ethyloleat und Ethyllaureat; Agar; Puffermittel wie Magnesiumhydroxid und Aluminiumhydroxid; Alginsäure; Pyrogen-freies Wasser; isotonische Salzlösung; Ringers Lösung, Ethylalkohol und Phosphatpufferlösungen wie auch andere nichttoxische kompatible Substanzen, die in pharmazeutischen Formulierungen verwendet werden. Benetzungsmittel, Emulgatoren und Gleitmittel wie Natriumlaurylsulfat und Magnesiumstearat, wie auch Färbemittel, Beschichtungsmittel sowie Parfümierungsmittel und Konservierungsstoffe können ebenso in den Zubereitungen vorhanden sein, entsprechend den Anforderungen des Galenikers. Die Menge des aktiven Wirkstoffes, der mit den Trägermaterialien kombiniert wird, um eine Einzeldosis zu produzieren, wird abhängig von dem behandelten Patienten und der speziellen Methode der Verabreichung variieren.

Pharmazeutisch annehmbare Salze der erfindungsgemäßen Aptamere können auf gut bekanntem Weg, beispielsweise durch Lösen der erfindungsgemäßen Verbindungen in der entsprechenden wässrigen Pufferlösungen oder $H_2O$ und anschließendem Gefriertrocknen hergestellt werden. Metallsalze können erhalten werden durch Lösen der erfindungsgemäßen Verbindungen in Lösungen, die das entsprechende Ion enthalten, und anschließendem Isolieren der Verbindung über HPLC oder Gelpermeationsverfahren.

Weitere Verfahren und Methoden zur Herstellung von Aptamermedikamenten sowie deren Verabreichung sind z.B. in der WO 02/26932 offenbart, und können selbstverständlich hier ebenfalls angewendet werden.

Die erfindungsgemäßen Nukleinsäuren können in einfacher Weise beispielsweise durch herkömmliche chemische Synthese mithilfe eines DNA/RNA-Synthesizer hergestellt werden, ohne den Fachmann vor irgendwelche Probleme zu stellen. Die entsprechenden Protokolle und Geräte sind auf dem Fachgebiet bestens bekannt und Routine.

Figur 1 zeigt eine für SEQ ID NO 2 postulierte Sekundärstruktur. Das Aptamer SEQ ID NO 2 bildet mit der RNA-Sequenz 5'-CGGAC GGGAC CAGAG GUGCC GCUGU CCG-3' die Struktur H1 aus. Dieser Bereich mit Helix-(Hairpin)-Struktur (H1) ist flankiert von zwei RNA-Abschnitten, die ebenfalls eine Helix-Struktur ausbilden können, genannt "Helix F". Die Synthese von veränderten/mutierten RNAs ausgehend von Aptamer 89 (SEQ ID NO 2) zeigt, ob die flankierenden Sequenzen, die die Helix F ausbilden, wichtig für die Funktion des Aptamers sind und ob das Aptamer 89 verkürzt werden kann, bei Erhalt seiner Funktion (SEQ ID NO 4, **Beispiel 7**).

Figur 2 zeigt eine für SEQ ID NO 7 errechnete Sekundärstruktur. Die Struktur weist im zentralen Bereich der Sequenz (SEQ ID NO 9) einen Hairpin Loop (H2) auf, der durch eine weitere Helix aus den flankierenden Sequenzen ergänzt wird.

Die nachfolgenden Beispiele erläutern die vorliegende Erfindung, ohne jedoch einschränkend zu sein.

## BEISPIELE

Die folgende Übersicht zeigt die verwendeten Materialien und Bezugsquellen:

| Material | Firma, Ort | Best.-Nr | Bemerkung |
|---|---|---|---|
| Phenol | Carl Roth, Karlsruhe | 0038.1 | TE-äquilibriert |
| T7-RNA Polymerase | MBI-Fermentas, St. Leon-Roth | EP0111 | |
| ATP, GTP | Carl Roth, Karlsruhe | K045.1<br>K047.1 | |
| 2'-$NH_2$-UTP<br>2'-$NH_2$-CTP | tebu-bio, Offenbach | N-1027<br>N-1026 | Hersteller: TriLink, San Diego |
| RQ1 DNase I, RNase frei | Promega, Mannheim | M6101 | |
| Chemikalien, Biochemikalien | Carl Roth, Karlsruhe<br>Merck, Darmstadt;<br>Sigma, Deisenhofen | | |

Bidestilliertes Wasser zum Ansetzen von Lösungen wurde mit Diethylpyrocarbonat (DEPC), 0,01 % für 24 Stunden bei 20 bis 25°C behandelt und anschließend für 60 min bei 121°C autoklaviert.

| **Lösungen:** | |
|---|---|
| CI | Chloroform/Isoamylalkohol-Mischung 24:1 (Volumen/Volumen) |
| 1xTBE | 90 mM Tris, 90 mM Borsäure, 2 mM EDTA |

**Beispiel 1**: Synthese der erfindungsgemäßen Aptamere

Aptamer bezeichnet eine modifizierte RNA, bei der die Pyrimidin-Nukleotide (U und C) an der 2'-Position statt der OH-Gruppe eine $NH_2$- (Amino-) Gruppe tragen. Die Synthese der modifizierten RNA erfolgt hier enzymatisch mittels einer RNA Polymerase durch Einbau von unmodifizierten Purin- und modifizierten Pyrimidin-Nukleotiden.

Ausgehend von doppelsträngiger DNA (60-70 pmol DNA), die die T7 Promotor-Sequenz des Bakteriophagen T7 und, ab dem ersten transkribierten Nukleotid eine DNA-Kopie der gewünschten Aptamer-RNA (z.B. für Aptamer Nr. 89: DNA-Sequenz "SEQ ID NO 1", beginnend mit 5'-GGGAGAC...-3', siehe **Beispiel 2**) enthält, werden die Aptamer-RNAs durch die T7 RNA Polymerase synthetisiert. Die Transkription erfolgt in Gegenwart von ATP und GTP sowie der modifizierten Nukleotide 2'-$NH_2$-UTP (2' $NH_2$-2'-desoxyuridin) und 2'- $NH_2$-CTP (2' $NH_2$-2'-desoxycytidin) durch Inkubation mit der käuflichen T7 RNA Polymerase in Gegenwart der vom Hersteller empfohlenen Puffer- und Salzbedingungen (Hersteller MBI-Fermentas, St. Leon-Roth) in einem Volumen von 40 µl bei 16°C +/- 3 °C für 20 bis 44 Stunden. Dann wird die noch vorhandene DNA durch Zusatz von $MgCl_2$ (Erhöhung der Konzentration um 2,74 mM) und RNase-freier DNase I (Endkonzentration 0,06 units/µl) und Inkubation bei 37°C für 135 min verdaut, wobei 1 unit DNase I definiert ist als die Enzymmenge, die 1 µg DNA in 50 µl in Puffer mit 40 mM Tris-HCl, 10 mM NaCl, 6 mM $MgCl_2$, 10 mM $CaCl_2$, (pH 7,9 bei 25°C), in 10 Minuten bei 37°C komplett verdaut.

Nach Zugabe von 45 µl TE-Puffer (10 mM Tris-HCl, 0,1 mM EDTA, pH 7,8) wird die Lösung zunächst mit Phenol und dann mit Chloroform/Isoamylalkohol (24:1) extrahiert. Danach wird die Aptamer-RNA mit Ethanol gefällt und nach Waschen mit 70 % Ethanol in 30 µl DEPC-behandeltem bidestilliertem $H_2O$ gelöst und bei -20°C aufbewahrt. Die Ausbeute an Aptamer - RNA wird durch Analyse der Extinktion bei 260 und 280 nm, sowie zusammen mit Nukleinsäuren bekannter Konzentration, nach Trennung in einem Polyacrylamid-Gel in Gegenwart von 8 M Harnstoff und Tris-Borsäure-Puffer mit 9,47 % (w/v) Acrylamid und 0,53 % Bis-Acrylamid, 8 M Harnstoff und TBE-Puffer (Standard 1xTBE-Puffersystem: 90 mM Tris, 90 mM Borsäure, 2 mM EDTA) bestimmt, die Trennung der Nukleinsäuren erfolgte bei 12 bis 16 V/cm für 35 bis 45 min. Das Gel wurde mit dem Fluoreszenzfarbstoff SYBRGreen II (Molecular Probes; Konzentration nach Herstellerangabe) für 20 min angefärbt, mit UV-Licht angeregt und die Fluoreszenz im sichtbaren Licht analysiert.

**Beispiel 2:** Sequenzen der erfindungemäßen Aptamere

1.) Sequenz der DNA Nr. 89 (SEQ ID NO 1)
5'-GGGAG ACGAT ATTCG TCCAT CACCG GACGG GACCA GAGGT GCCGC TGTCC GACTG AATTC TCGAC C-3'

2.) RNA-Sequenz des Pyrimidin-modifizierten Aptamers Nr. 89 (SEQ ID NO 2)
(U = 2'-$NH_2$-2'-desoxyuridin; C = 2'-$NH_2$-2'-desoxycytidin)
5'-GGGAG ACGAU AUUCG UCCAU CACCG GACGG GACCA GAGGU GCCGC UGUCC GACUG AAUUC UCGAC C-3'

3.) RNA-Sequenz des Kernbereichs des Pyrimidin-modifizierten Aptamers Nr. 89 ohne flankierende Sequenzen (SEQ ID NO 3)
(U = 2'-$NH_2$-2'-desoxyuridin; C = 2'-$NH_2$-2'-desoxycytidin)
5'-ACCGG ACGGG ACCAG AGGUG C-3'

4.) RNA-Sequenz der Aptamer-Mutante Nr. 89-2 (58 nt) (SEQ ID NO 4)
(U = 2'-$NH_2$-2'-desoxyuridin; C = 2'-$NH_2$-2'-desoxycytidin)
5'-GGGAG ACGAU AUUCG UCCAU CACCG GACGG GACCA GAGGU GCCGC UGUCC GACAU GGA-3'

5.) RNA-Sequenz der Aptamer-Mutante Nr. 89-Z (30 nt) (SEQ ID NO 5)
(U = 2'-$NH_2$)-2'-desoxyuridin; C = 2'-$NH_2$-2'-desoxycytidin)
5'-ACCGG ACGGG ACCAG AGGUG CCGCU GUCCG-3'

6.) Sequenz der DNA Nr. 82 (SEQ ID NO 6)
5'-GGGAG ACGAT ATTCG TCCAT CGGGA GCAGG GAAGG GGGCC GCTGT CCGAC TGAAT TCTCG ACC-3'

7.) RNA-Sequenz des Pyrimidin-modifizierten Aptamers Nr. 82 (SEQ ID NO 7)
(U = 2'-$NH_2$-2'-desoxyuridin; C = 2'-$NH_2$-2'-desoxycytidin)
5'-GGGAG ACGAU AUUCG UCCAU CGGGA GCAGG GAAGG GGGCC GCUGU CCGAC UGAAU UCUCG ACC-3'

8.) RNA-Sequenz des Kernbereichs des Pyrimidin-modifizierten Aptamers Nr. 82 ohne flankierende Sequenzen (SEQ ID NO 8)
(U = 2'-$NH_2$-2'-desoxyuridin; C = 2'-$NH_2$-2'-desoxycytidin)
5'-GGGAG CAGGG AAGGG GGC-3'

9.) RNA-Sequenz der Aptamer-Mutante Nr. 82-Z (30 nt) (SEQ ID NO 9)
(U = 2'-$NH_2$-2'-desoxyuridin; C = 2'-$NH_2$-2'-desoxycytidin)
5'-UCGGG AGCAG GGAAG GGGGC CGCUG UCCGA-3'

**BEISPIEL 3**: Kultivierung von humanen Endothelzellen aus Nabelschnur-Venen (HUVEC)

**Lösungen (steril) :**

Kulturmedium: IF-Basalmedium + 15% (v/v) NCS, 5 μg/ml Transferrin, 5 μg/ml Heparin, 0,7 μg/ml FGF, 2 mM L-Glutamin [IF-Basalmedium: 1:1-Mischung aus Iscove's Modifiziertem Dulbecco Medium (IMDM) und Ham's F12, beide von Life Technologies, Paisley (England)]

NCS: Serum neugeborener Kälber (Sebak, Aidenbach)

FGF: Fibroblastenwachstumsfaktor (eigene Herstellung, partiell aufgereinigt aus Schweinehirn)

**Materialien :**

Zellkulturgefäße, gelatiniert

**Durchführung :**

Die Kultivierung von HUVEC erfolgt in gelatinebeschichteten Kulturgefäßen bei 37°C, 5% $CO_2$ und Wasserdampf-gesättigter Luftatmosphäre. Das Kulturmedium wird alle 2-3 Tage gewechselt; bei Konfluenz werden die Zellen mit einer Teilungsrate von 1:3 bis 1:5 passagiert. HUVEC wachsen streng kontaktinhibiert und bilden einschichtige Zellrasen mit der typischen Kopfsteinpflastermorphologie. Bei Konfluenz erreichen die Kulturen Zelldichten von 4-9 x $10^4$ Zellen/$cm^2$. Für Apoptoseuntersuchungen werden ausschließlich HUVEC-Kulturen der Passagen 1-4 verwendet.

**Beschichtung von Kulturgefäßen:**

Lösungen (steril) :

Gelatinelösung, 1% (w/v) in Milli-Q-Wasser

1 g Gelatine (zellkulturgetestet) in 100 ml Milli-Q-Wasser suspendieren, durch Autoklavieren für 20 min bei 121°C und 2 bar lösen und bei Raumtemperatur lagern.

PBS (140 mM NaCl, 3 mM KCl, 8 mM $Na_2HPO_4$, 1,5 mM $KH_2PO_4$)

8 g/l NaCl

0,2 g/l KCl

1,44 g/l $Na_2HPO_4$ x 2 $H_20$

0,2 g/l $KH_2PO_4$

Die Salze in einem entsprechenden Volumen Milli-Q-Wasser lösen, 20 min bei 121°C und 2 bar autoklavieren und bei Raumtemperatur lagern. Der pH-Wert wird überprüft und liegt zwischen 7,2 und 7,4.

**Materialien :**

Zellkulturgefäße

**Durchführung :**

Für die Kultivierung adhärent wachsender Zellen werden Kulturgefäße mit Gelatine beschichtet. Der Boden der Zellkulturgefäße wird mit steriler Gelatinelösung bedeckt, die Zellkulturgefäße werden 15 min bei Raumtemperatur belassen. Die Gelatinelösung wird abgesaugt, die Zellkulturgefäße werden einmal mit PBS gewaschen und können so verwendet werden.

**Subkultivierung adhärenter Zellen**

Lösungen (steril):

PBS

Trypsin/EDTA (0,05% (w/v)/0,02% (w/v))

0,1 ml Trypsin-Stammlösung

0,05 ml EDTA-Stammlösung

Mit sterilem PBS auf 50 ml auffüllen, und in Portionen zu 10 ml bei -20°C lagern.

**Materialien:**

Zellkulturgefäße, gelatiniert

**Durchführung:**

Alle aufgeführten Zelltypen werden mit Trypsin/EDTA-Lösung von der Kulturfläche abgelöst. Das Kulturmedium wird abgesaugt, der Boden des Kulturgefäßes kurz mit PBS gewaschen und mit Trypsin/EDTA-Lösung (~1 ml für eine 25 $cm^2$-Kultur-flasche) bedeckt. Die Enzymlösung wird sofort wieder abgesaugt, so dass ein dünner Flüssigkeitsfilm auf den Zellen verbleibt. Die Zellen werden 1-10 min bei Raumtemperatur belassen und das Ablösen der Zellen unter dem Mikroskop verfolgt. Das Ablösen der Zellen kann durch sanftes Aufschlagen des Kulturgefäßes auf der Kante beschleunigt werden. Die Zellen werden in frischem Kulturmedium aufgenommen, eventuell gezählt und in neue Kulturgefäße ausgesät.

**BEISPIEL 4**: Bestimmung der Apoptoserate durch Färbung apoptotischer Zellen mit DAPI

DAPI gehört zur Indolfarbstoffgruppe und ist ein selektiver DNS-Farbstoff. Der Farbstoff wird bei 340-360 nm angeregt, und das Emissionsmaximum liegt bei 480 nm. Er wird für Apoptoseuntersuchungen eingesetzt [ vgl. Cohen et al., Immunology Today, 14, NO. 3, 126-130 (1993)].

**Morphologische Auswertung:**

Lösungen:

PBS

Formaldehydlösung

4% (v/v) Formaldehyd in PBS

DAPI-Lösung (Molecular Probes, Leiden, Niederlande)

2 $\mu$g/ml DAPI in Methanol

**Materialien:**

Petrischale (35 mm) oder 24-Loch Platte mit HCTVEC-Zellen in Kultur

**Durchführung:**

Der Kulturüberstand einer Petrischale oder 24 Loch Platte wird abgesaugt, der Zellrasen wird 15 Minuten mit 1 ml Formaldehydlösung auf Eis fixiert, zweimal mit 2 ml PBS gewaschen, für 15 Minuten mit 0,5 ml DAPI-Lösung versetzt, mit PBS gewaschen und unter dem Fluoreszenzmikroskop ausgewertet.

Es wird mit dem UV-Filtersatz und einem 20 x oder 40 x Objektiv gearbeitet. 500-1000 Zellen werden zufällig ausgewählt und die Zellen mit apoptotischen Kernen gezählt.

Der Apoptose-Index wird nach folgender Formel berechnet:

$$\text{Apoptose-Index [\%]} = \text{Anzahl apoptotischer Zellen/Gesamtzellzahl} \times 100$$

**BEISPIEL 5**: Testsystem für anti-apoptotisch wirksame Aptamere

Die Zellen werden wie in **Beispiel 3** beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den eigentlichen Test eingesetzt.

Die Induktion der Apoptose erfolgt durch das von den Endothelzellen selbst produzierte und sekretierte TSP-1, welches sich im Kulturmedium anreichert (Auto-Konditionierung des Kulturmediums).

Es werden Untersuchungen zum Einfluss der verschiedenen Aptamere auf die Apoptoserate von Endothelzellen durchgeführt. Hierfür werden die Aptamere, die in DEPC-behandeltem bidestillierten Wasser gelöst sind (**Beispiel 1**) in dem Kulturmedium für HUVEC (**Beispiel 3**) verdünnt und in den angegebenen Konzentrationen eingesetzt. Als Positivkontrolle wird Kulturmedium ohne jegliche Aptamere oder Inhibitoren eingesetzt.

Das Medium mit den Aptameren wird zu den Zellen gegeben und für drei Tage unter Kulturbedingungen (**Beispiel 3**) inkubiert. Nach 36 Stunden wird das Kulturmedium/ Kulturmedium mit Aptameren einmal gewechselt.

Anschließend werden die Zellen wie in **Beispiel 4** beschrieben zur Bestimmung der Apoptoserate mit DAPI gefärbt und der Apoptose-Index nach der angegebenen Formel bestimmt.

Die deutliche Apoptose-induzierende Wirkung des auto-konditionierten Mediums im Falle der Positivkontrolle und die reduzierte Apoptose im Falle der Inhibitionskontrolle zeigen als interne Kontrollen ein Gelingen des Testes an.

**BEISPIEL 6**: Identifikation von anti-apoptotisch wirksamen Aptameren mit Hilfe des erfindungsgemäßen Verfahrens

Die Zellen werden wie in **Beispiel 3** beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den Test nach Beispiel 4 eingesetzt. Folgende Proben werden angesetzt:

(K) Kulturmedium [auto-konditioniertes Medium, Basisrate der Apoptose, Kontrolle],

(1) Kulturmedium + Aptamer 89, SEQ ID NO 2, Konzentration: 150 nM

(2) Kulturmedium + Aptamer 89, SEQ ID NO 2, Konzentration: 300 nM

(3) Kulturmedium + Aptamer 82, SEQ ID NO 7, Konzentration: 150 nM

(4) Kulturmedium + Aptamer 82, SEQ ID NO 7, Konzentration: 300 nM

Nach 72 h Inkubation unter Kulturbedingungen werden die Zellen fixiert, mit DAPI gefärbt und morphologisch unter dem Fluoreszenzmikroskop untersucht. Die apoptotischen Zellen und die Gesamtzellzahl werden bestimmt und der Apoptoseindex berechnet (Prozent apoptotischer Zellen) (**Beispiel 3, Beispiel 4, Beispiel 5**).

**Tabelle 1:** Es werden folgende Aptamere getestet. Die dazugehörenden Sequenzen sind in Beispiel 2 angegeben:

| PROBE NO | Aptamer-Bezeichnung, Konzentration | Apoptose -index [%] | Inhibitions -index [%]* |
|---|---|---|---|
| K | Kontrolle | 3,59 ± 0,54 | -- |
| (1) | 89, 150 nM | 0,17 ± 0,15 | 95,24 |
| (2) | 89, 300 nM | 0,16 ± 0,14 | 95,65 |
| (3) | 82, 150 nM | 1,22 ± 0,27 | 66,03 |
| (4) | 82, 300 nM | 0,74 ± 0,19 | 79,32 |
| * 100% = keine Apoptose mehr detektierbar; 0% = keine Wirkung = Kontrolle | | | |

**BEISPIEL 7**: Identifikation der minimierten Sequenz der anti-apoptotisch wirksamen Aptamere durch Verkürzung

Der Test wurde wie in **Beispiel 6** beschrieben durchgeführt. Dabei wurden ein Aptamer mit der reduzierten Gesamtlänge (SEQ ID NO 4, hier genannt "89-2") mit dem Aptamer 89 (volle Länge SEQ ID NO 2, genannt "89") verglichen. Auch bei SEQ ID NO 4 bildet sich - wie in Figur 1 dargestellt - die thermodynamisch bevorzugte Hairpin-Struktur H1 aus. Dagegen wurde die bei SEQ ID NO 2 am 3'-Ende vorhandene Sequenz 5'-UGAAUUCUCGACC-3' bei SEQ ID NO 4 ersetzt durch die RNA-Sequenz 5'-AUGGA-3' (modifizierte RNA, siehe **Beispiel 2**). Dadurch wird die Struktur der "Helix F" zerstört, und es kann eine neue kurze Helix entstehen (5'-AUGGA/5'-UCCAU).

Die Zellen werden wie in **Beispiel 3** beschrieben kultiviert. Die Zellen werden in die entsprechenden Kulturgefäße (z.B. 24-Lochplatte/0,5 ml pro Kavität) ausgesät und nach dem Erreichen der vollständigen Konfluenz für den Test eingesetzt (**Beispiel 4, Beispiel 5**). Folgende Proben werden angesetzt:

(K) Kulturmedium [auto-konditioniertes Medium, Basisrate der Apoptose, Kontrolle],

(5) Kulturmedium + Aptamer 89, SEQ ID NO 2, Konzentration: 50 nM

(6) Kulturmedium + Aptamer 89,-2 SEQ ID NO 4, Konzentration: 50 nM

Die folgende Tabelle 2 faßt die Ergebnisse zusammen. Es ist deutlich zu erkennen, daß der verkürzte Sequenzbereich in Nr. 89-2 (SEQ ID NO 4) eine zur Ursprungssequenz Nr. 89 (SEQ ID NO 2) vergleichbare Aktivität aufweist.

**Tabelle 2:** Es werden folgende Aptamere getestet. Die dazugehörenden Sequenzen sind in Beispiel 2 angegeben:

| PROBE NO | Aptamer-Bezeichnung | Apoptose -index [%] | Inhibitions -index [%]* |
|---|---|---|---|
| K | Kontrolle | 4,99 ± 0,59 | -- |
| (5) | 89 | 0,44 ± 0,21 | 91,15 |
| (6) | 89-2 | 0,81 ± 0,30 | 83,76 |
| * 100% = keine Apoptose mehr detektierbar; 0% = keine Wirkung = Kontrolle; | | | |

## Patentansprüche

1. Nukleinsäure umfassend eine Nukleinsäuresequenz ausgewählt aus der Gruppe bestehend aus: SEQ ID NO 1 bis SEQ ID NO 9 und Varianten hierzu die mindestens 95% Sequenzidentität zu den Sequenzen ausgewählt aus der Gruppe bestehend aus: SEQ ID NO 1 bis SEQ ID NO 9 aufweisen und antiapoptotisch wirksam sind.

2. Nukleinsäure nach Anspruch 1 **dadurch gekennzeichnet, dass** sich an die Sequenz ausgewählt aus der Gruppe bestehend aus: SEQ ID NO 1 bis SEQ ID NO 9 in 5'-und/oder 3 '-Richtung nicht mehr als 100, bevorzugt nicht mehr als 70, besonders bevorzugt nicht mehr als 30 Nukleotide, ganz besonders bevorzugt nicht mehr als 20, und am meisten bevorzugt nicht mehr als 10 Nukleotide anschließen.

3. Nukleinsäure ausgewählt aus der Gruppe bestehend aus SEQ ID NO 1 bis SEQ ID NO 9.

4. Pharmazeutische Zubereitung umfassend eine Nukleinsäure nach einem der Ansprüche 1 bis 3.

5. Verwendung der Nukleinsäuren nach Anspruch 1 bis 3 als Diagnosetool.

6. Verfahren zur Herstellung der Nukleinsäuren nach Anspruch 1 bis 3 durch chemische Synthese.

## Claims

1. Nucleic acid comprising a nucleic acid sequence selected from the group consisting of: SEQ ID NO 1 to SEQ ID NO 9, and variants thereof which exhibit at least a 95 % sequence identity to the sequences selected from the group consisting of SEQ ID NO 1 to SEQ ID NO 9 and which are antiapoptotically active.

2. Nucleic acid, according to Claim 1, **characterized in that** no more than 100, preferably no more than 70, especially preferably no more than 30, more especially preferably no more than 20 and most preferably of all no more than 10 nucleotides attach in 5' and/or 3' direction to the sequence selected from the group consisting of: SEQ ID NO 1 to SEQ ID NO 9.

**3.** Nucleic acid selected from the group consisting of SEQ ID NO 1 to SEQ ID NO 9.

**4.** Pharmaceutical preparation comprising a nucleic acid according to one of the Claims 1 to 3.

**5.** Use of the nucleic acids according to Claims 1 to 3 as a diagnostic tool.

**6.** Method of manufacturing the nucleic acids according to Claims 1 to 3 by chemical synthesis.

**Revendications**

**1.** Acide nucléique incluant une séquence d'acide nucléique choisie parmi le groupe constitué par: SEQ ID NO 1 à SEQ ID NO 9 et des variantes afférentes qui présentent une identité de séquence d'au moins 95% vis-à-vis des séquences choisies parmi le groupe constitué par SEQ ID NO 1 à SEQ ID NO 9 et qui présentent une activité anti-apoptotique.

**2.** Acide nucléique selon la revendication 1, **caractérisé en ce que** la séquence choisie parmi le groupe constitué par: SEQ ID NO 1 à SEQ ID NO 9 est suivie dans la direction 5' et/ou 3' par pas plus de 100, de préférence pas plus de 70, de façon particulièrement préférable pas plus de 30 nucléotides, de façon très particulièrement préférable pas plus de 20 et de la façon la plus particulièrement préférable pas plus de 10 nucléotides.

**3.** Acide nucléique choisi parmi le groupe constitué par: SEQ ID NO 1 à SEQ ID NO 9.

**4.** Composition pharmaceutique comprenant un acide nucléique selon l'une des revendications 1 à 3.

**5.** Utilisation des acides nucléiques selon les revendications 1 à 3 en tant qu'outil de diagnostic.

**6.** Procédé pour la préparation des acides nucléiques selon les revendications 1 à 3 par synthèse chimique.

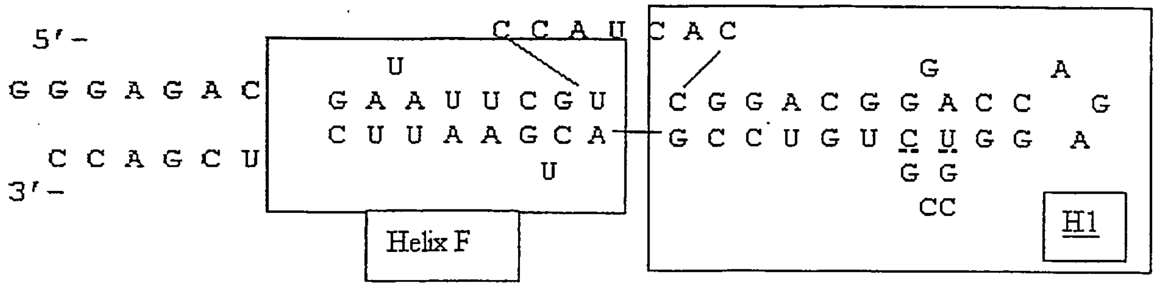


**Figur 1**

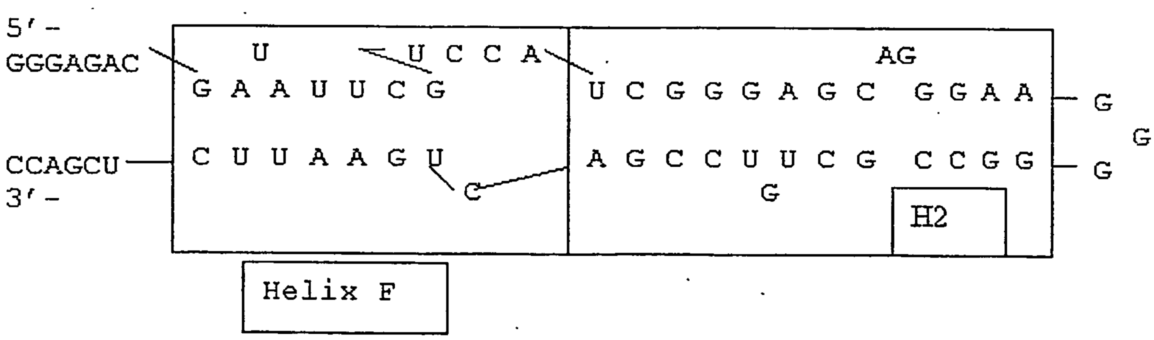


**Figur 2**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 02083160 A
- DE 10163130
- WO 0226932 A


### In der Beschreibung aufgeführte Nicht-Patentliteratur


- **GOLD.** *Annu. Rev. Biochem.,* 1995, vol. 64, 763-797
- **ELLINGTON ; CONRAD.** *Biotechnol. Annu. Rev.,* 1995, vol. 1, 185-214
- **FAMULOK.** *Curr. Opin. Struct. Biol.,* 1999, vol. 9, 324-329
- **OSBORNE, S. E. ; A. D. ELLINGTON.** Nucleic Acid Selection and the Challenge of Combinatorial Chemistry. *Chem. Rev,* 1997, vol. 97 (2), 349-370
- **JAYASENA, S. D.** Aptamers: an emerging class of molecules that rival antibodies in diagnostics. *Clin Chem,* 1999, vol. 45 (9), 1628-1650
- **CERCHIA, L. ; J. HAMM et al.** Nucleic acid aptamers in cancer medicine. *FEBS Lett,* 2002, vol. 528 (1-3), 12-16
- **KÖHLER, G. ; C. MILSTEIN.** Continuous cultures of fused cells secreting antibody of predefined specificity. *Nature,* 1975, vol. 256 (5517), 495-497
- **AGRAWAL, S.** Antisense oligonucleotides: towards clinical trials. *Trends Biotechnol,* 1996, vol. 14 (10), 376-387
- **PIEKEN, W. A. ; OLSEN D. B. et al.** Kinetic characterization of ribonuclease-resistant 2'-modified hammerhead ribozymes. *Science,* 1991, vol. 253 (5017), 314-317
- **RUCKMAN, J. ; GREEN L. S. et al.** 2'-Fluoropyrimidine RNA-based aptamers to the 165-amino acid form of vascular endothelial growth factor (VEGF165). Inhibition of receptor binding and VEGFinduced vascular permeability through interactions requiring the exon 7-encoded domain. *J Biol Chem,* 1998, vol. 273 (32), 20556-20567
- Preclinical and phase 1A clinical evaluation of an anti-VEGF pegylated aptamer (EYE001) for the treatment of exudative agerelated macular degeneration. *Eyetech Study Group,* 2002, vol. 22 (2), 143-152
- **FREYBERG et al.** *BBRC,* 2001, vol. 286, 141-149
- **COHEN et al.** *Immunology Today,* 1993, vol. 14 (3), 126-130

SEQUENZPROTOKOLL

<110> CytoTools GmbH

<120> Anti-apoptotisch wirksame Aptamere

<130> C 720

<160> 9

<170> Patent In version 3.1

<210> 1
<211> 66
<212> DNA
<213> Künstliche Sequenz

<220>
<223> Aptamer

<400> 1

```
gggagacgat attcgtccat caccggacgg gaccagaggt gccgctgtcc gactgaattc     60

tcgacc                                                                66
```

<210> 2
<211> 66
<212> RNA
<213> Künstliche Sequenz

<220>
<221> modifizierte Base
<222> (1) .. (66)
<223> Aptamer; U = 2'-NH2-2'-desoxyuridin; C = 2'-NH2-2'-desoxycytidin

<400> 2

```
gggagacgau auucguccau caccggacgg gaccagaggu gccgcugucc gacugaauuc     60

ucgacc                                                                66
```

<210> 3
<211> 21
<212> RNA
<213> Künstliche Sequenz

<220>
<221> modifizierte Base
<222> (1) .. (21)
<223> <220>
<223> Aptamer; U = 2'-NH2-2'-desoxyuridin; C = 2'-NH2-2'-desoxycytidin

<400> 3

accggacggg accagaggug c 21

<210> 4
<211> 58
<212> RNA
<213> Künstliche Sequenz

<220>
<221> modifizierte Base
<222> (1) .. (58)
<223> Aptamer; U = 2'-NH2-2'-desoxyuridin; C = 2'-NH2-2'-desoxycytidin

<400> 4
gggagacgau auucguccau caccggacgg gaccagaggu gccgcugucc gacaugga 58

<210> 5
<211> 30
<212> RNA
<213> Künstliche Sequenz

<220>
<221> modifizierte Base
<222> (1)..(30)
<223> Aptamer; U = 2'-NH2-2'-desoxyuridin; C = 2'-NH2-2'-desoxycytidin

<400> 5
accggacggg accagaggug ccgcuguccg 30

<210> 6
<211> 63
<212> DNA
<213> Künstliche Sequenz

<220>
<223> Aptamer

<400> 6

gggagacgat attcgtccat cgggagcagg gaagggggcc gctgtccgac tgaattctcg 60

acc 63

<210> 7
<211> 63
<212> RNA
<213> Künstliche Sequenz

<220>
<221> modifizierte Base
<222> (1)..(63)
<223> Aptamer; U = 2'-NH2-2'-desoxyuridin; C = 2'-NH2-2'-desoxycytidin

<400> 7

gggagacgau auucguccau cgggagcagg gaagggggcc gcuguccgac ugaauucucg 60

acc 63

<210> 8
<211> 18
<212> RNA
<213> Künstliche Sequenz

<220>
<221> modifizierte Base
<222> (1)..(18)
<223> Aptamer; U = 2'-NH2-2'-desoxyuridin; C = 2'-NH2-2'-desoxycytidin

<400> 8
gggagcaggg aagggggc 18

<210> 9
<211> 30
<212> RNA
<213> Künstliche Sequenz

<220>
<221> modifizierte Base
<222> (1)..(30)
<223> Aptamer; U = 2'-NH2-2'-desoxyuridin; C = 2'-NH2-2'-desoxycytidin

<400> 9
ucgggagcag ggaagggggc cgcuguccga 30